# EUROPEAN PATENT APPLICATION

(11) **EP 2 878 679 A1**
(43) Date of publication of application: **03.06.2015**
(21) Application number: 13822557.8
(22) Date of filing: 20.05.2013
(51) Int. Cl.: C12Q 1/68, C12N 15/11, C12N 5/00

(54) **METHOD FOR NONDESTRUCTIVE DETECTION OF miRNA EXPRESSION IN CELL AND DETERMINATION OF CELL TYPE AND STATE**

(30) Priority: 24.07.2012 CN 201210258472
(71) Applicant: Institute Of Zoology, Chinese Academy Of Sciences, Chaoyang District, Beijing 100101 (CN); Institute Of Genetics And Developmental Biology, Chinese Academy Of Sciences, Beijing 100101 (CN)
(72) Inventor: ZHOU, Qi, Beijing 100101 (CN); WANG, Xiujie, Beijing 100101 (CN); ZHANG, Ying, Beijing 100101 (CN)
(74) Representative: Potter Clarkson LLP
(86) International application number: PCT/CN2013/075903
(87) International publication number: WO 2014/015706

(57) **Abstract**

The present invention provides a method for nondestructive detection of a miRNA expression in a cell and determination of a cell type and state, and specifically provides a method for determining a cell type and state according to a miRNA expression in a cell culture medium. The method comprises: culturing cells of different types, collecting a culture medium of the cells, extracting RNA in the culture medium, performing inverse transcription on the RNA, detecting miRNA in the cell culture medium by using a fluorescent quantitative PCR method, and determining the type and state of the detected cell according to a relationship between miRNA expressions of different cell types and states and the detected miRNA expression. The method of the present invention proves for the first time that the miRNA expression in the culture medium can be detected to determine the type and state of a cell, comprising the pluripotency level of a stem cell and the state of a cell obtained through transdifferentiation, which avoids causing damages to the cell and is especially suitable for experimental and clinical applications where the number of cells is limited.

## Description

### Technical Field

The invention belongs to the biotechnology field, and relates to a method to detect expression level of microRNA (miRNA) in cell and determine the cell type and cell status. In particular, the invention relates to a fast, convenient, non-invasive and continuous method for detecting the pluripotency levels of stem cells, or identifying the cell type and cell status during the cell culture period. Specifically, the present invention relates to a method for determining the type and status of cells according to the expression level of the microRNAs (miRNAs) in a cell culture medium, which comprises culturing various types of cells, collecting the media of the cells, then performing a reverse transcription on the extracted RNAs from the media, detecting the miRNA in the cell media by the fluorescence quantitative PCR, and thereby determining the pluripotency levels of stem cells or identifying the cell type and status depending on the expression level of specific miRNAs.

### Background Art

MiRNAs are non-coding microRNAs with lengths of about 21-25 nucleotides (nt) which widely exist in eukaryote genomes. MiRNAs are generally transcribed by miRNA genes located in intergenic regions and introns to generate primary miRNAs (pri-miRNAs). The pri-miRNAs are then processed in nucleus into precursor miRNAs (pre-miRNAs) with lengths about 70 nt, which are then transported to cytoplasm and processed into mature miRNAs. Mature miRNAs are introduced into miRNA-induced gene silencing complex (miRISC), and then pair with target mRNAs. MiRNAs negatively regulate gene expression by degradation of target mRNAs or blockage of protein translation (Valencia-Sanchez, M.A., et al., Control of translation and mRNA degradation by miRNAs and siRNAs. Genes Dev, 2006. 20(5): p. 515-24). Recent studies have found that there are over 1900 types of miRNAs in human, which participate in the expression regulation of about one-third of human genes involving in several critical life processes.

In 2008, Chen et al. had demonstrated that a large amount of miRNAs can be detected in serum of many species, such as human beings, rats, mice, calves, horses and the like, by Solexa sequencing technology (Chen, X., et al., Characterization of microRNAs in serum: a novel class of biomarkers for diagnosis of cancer and other diseases. Cell Res, 2008. 18(10): p. 997-1006). Thereafter, the results of many studies demonstrated that the miRNAs in serum are related to the expression profiles of the miRNAs in tumour tissues (Ng, E.K., et al., Differential expression of microRNAs in plasma of patients with colorectal cancer: a potential marker for colorectal cancer screening. Gut, 2009. 58(10): p. 1375-81; Lawrie, C.H., et al., Detection of elevated levels of tumour-associated microRNAs in serum of patients with diffuse large B-cell lymphoma. Br J Haematol, 2008. 141(5): p. 672-5; Lawrie, C.H., et al., MicroRNA expression distinguishes between germinal center B cell-like and activated B cell-like subtypes of diffuse large B cell lymphoma. Int J Cancer, 2007. 121(5): p. 1156-61; Zhu, W., et al., Circulating microRNAs in breast cancer and healthy subjects. BMC Res Notes, 2009. 2: p. 89). Furthermore, Gilad et al. found that quantitative real-time RT-PCR (qRT-PCR) technology can be used to quantify the miRNAs in urine, saliva, amniotic fluid and pleural fluid of human body (Gilad, S., et al., Serum microRNAs are promising novel biomarkers. PLoS One, 2008. 3(9): p. e3148). These findings pave a new way for studying miRNAs, demonstrating that miRNAs are extensively contained in body fluids.

Currently, the methods for studying miRNAs in cells are generally carried out by collecting destructed cells and then extracting RNAs therefrom. However, since the cells of clinical application value, such as embryonic stem cells, induced pluripotent stem cells (iPS cells) are generally limited in number, and the characters of the cells, such as the property and status need to be monitored dynamically prior to clinical application. Collecting destructive cells will cause not only a large number of cell loss, but also the detection results are not consistent with those obtained from the continual cultured cells, which is a major bottle-neck for the cells with limited number in fundamental research and clinical application. Regarding this problem, the present invention proves for the first time that there are detectable miRNAs in cell culture media, and the variation tendency of their relative expression abundance is consistent with that of the miRNAs in cells. As for detection methods, in the present invention, the TRIZOL LS is used to extract miRNAs, and glycogen is used to make RNAs visible so as to prevent the loss of RNAs in maximum extent.

Cell type transformation is an important direction for cell biology study (such as transformation of differentiated animal adult cells into iPS cells and transdifferentiation between various types of adult cells) by different ways, which also holds large potential in clinical application. However, the induction cycles of the current methods for cell fate transformation are generally long, and the successful rates are very low. If the status of cells can be detected dynamically during the induction period, the properties of cells would be determined at early stage of the induction, which are advantageous to quickly screen out target cells meeting the requirements, and are of great values in fundamental researches and clinical applications. In the present invention, we demonstrate that the expression of miRNAs in cell media is consistent with the type and status of cells, by continuously detecting the change of the miRNA expression in cell media every day during the induction of mouse embryo fibroblast (MEF) cells into iPS cells. Therefore, the status of cells can be determined and the transformation of cell types can be monitored dynamically by detecting the miRNAs expression in cell media.

### Disclosure of the Invention

The object of the present invention is to provide a method for detecting the contents of miRNAs in a cell medium to overcome the drawback in the prior arts. It is proved for the first time that the variation tendency of the relative abundance of miRNAs in cell media is consistent with that of miRNAs in cultured cells. Therefore, the types and status of cells can be identified by detecting the expression of miRNAs in the cell media so as to avoid to damage cells. The method is especially suitable for the fundamental researches and clinical applications in which the number of cells is limited. The invention further provides the detection results of miRNA expression in the culture media for pluripotent stem cells with different developmental potential (2N-iPS cells are partially pluripotent cells, and 4N-iPS cells are fully pluripotent cells) and their original cells, which proves that the relative expression level of specific miRNAs in media can indicate the pluripotency level of cells, wherein 2N-iPS cell lines IP20D-3 and IP36D-3, 4N-iPS cell lines IP14D-1 and IP14D-6 are all derived from mouse embryo fibroblast (MEF); 4N-iPS cell IP16DT-2A are derived from mouse tail-tip fibroblast (TTF); 4N-iPS cell IP14DN-5 are derived from mouse neuronal precursor cell (mouse AM1-3 cell).

Unless indicated specifically, "Ct value" used herein means "the number of accomplished cycles when the fluorescence signal in each reaction tube arrives at the set threshold".

Unless indicated specifically, "FBS" used herein means "fetal bovine serum".

Unless indicated specifically, "EGF" used herein means "epidermal growth factor".

Unless indicated specifically, "bFGF" used herein means "basic fibroblast growth factor".

Regarding above objects, the present invention provides technical solutions as follows:
In one aspect, the present invention provides a fast, convenient, and non-invasive method for detecting the expression level of miRNAs in cells and determining the types and status of the cells. The method is performed by detecting the expression levels of miRNAs in the media. The detection of the expression level of miRNAs in cell media is performed by a method comprising the following steps:
   1) collecting the media in which the cells have been cultured overnight, centrifuging the collected media to get the supernatant, and filtering the supernatant;
   2) extracting RNAs from the filtered supernatant obtained in step 1);
   3) performing a reverse transcription reaction on the RNAs extracted in step 2) to obtain a cDNA solution;
   4) performing a PCR amplification reaction on the cDNA solution obtained in step 3), and then detecting the PCR products by the fluorescence quantitative PCR instrument to obtain the expression level of miRNAs in the media;
   5) determining the change degree of the expression levels of miRNAs in cells according to the change degree of the expression levels of miRNAs in the media;

Preferably, the changing curve of the expression levels of miRNAs in the mediums is plotted to calculate the amount of miRNAs in cells.

Preferably, the medium in which the cells have been cultured overnight are the medium that the cells have been cultured for 3-16 days.

Preferably, when the expression levels of miRNAs in cells are detected, the cells are those during iPS induction. The cells are cultured, and the expression levels of miRNAs in the media are detected. The expression levels of miRNAs in cells are determined according to the equation log₂E cell = 1.5268× log₂E_{medium} + 3.1026. Preferably, the cells are those that have been cultured for 0-16 days; more preferably, the cells are those that have been cultured for 3-16 days.

The iPS cells are cultured from day 0 to day 16. The expression levels of miRNAs in cells and in the medium during the culture are measured, respectively. The correlation analysis is conducted on the detection results obtained from the cells and the media to obtain a regression equation for further statistical analysis: log₂E_{cell} = 1.5268 × log₂E_{medium} + 3.1026, wherein R²=0.8539. The analysis result demonstrates that above 85% of the data from the detection results in cells and the detection results in the media can be described with the regression equation. Then the corresponding quantity of miRNAs in cells can be determined according to this correlation from the quantity of miRNAs in the media in step 4).

Preferably, in step 1), the cells are one or more selected from the group consisting of human or animal embryo fibroblast, human or animal induced pluripotent stem cell (iPS cell), human or animal tail-tip fibroblast, animal embryonic stem cell (ES cell), human or animal adult stem cell, human or animal cell derived by transdifferentiation, differentiated cell of human or animal, or diseased cell such as tumor cell or diseased stem cell and the like.

Preferably, the animal embryo fibroblast can be mouse embryo fibroblast (MEF).

Preferably, the animal induced pluripotent stem cell can be selected from 2N-iPS cell and 4N-iPS cell. More preferably, 2N-iPS cell comprises the cell lines IP20D-3 and IP36D-3; 4N-iPS cell comprises the cell lines IP14D-1, IP 14D-6, IP16DT-2A and IP 14DN-5.

Preferably, the animal embryonic stem cell is mouse embryonic stem cell ESC2 and/or CL11.

Preferably, the differentiated cell of human or animal can be selected from animal tail-tip cell and/or mouse neuronal precursor cell.

Preferably, the animal tail-tip fibroblast can be mouse tail-tip fibroblast (TTF).

Preferably, the neuronal precursor cell can be mouse neuronal precursor cell (mouse AM1-3 cell).

Preferably, the tumors cell can be human breast cancer cell (MCF-7).

Preferably, when the cell is MEF, TTF or MCF-7 cell, the medium for culturing cell is the 450 ml DMEM medium supplemented with 50 ml FBS, 5 ml 100×mycillin.

Preferably, when the cell is AM1-3, the medium for culturing cell is the N2B27 medium supplemented with EGF and bFGF (their concentrations are all 20 ng/ml).

Preferably, when the cell is iPS cell (induced pluripotent stem cell), the medium for culturing cell is the DMEM/F12 containing 20% KOSR (serum substitute, Gibco) supplemented with 1000 U LIF (leukaemia inhibitory factor, Chemicon), 2 mM glutamine (Sigma), 1 mM sodium pyruvate (Sigma), and 0.1 mM β-mercaptoethanol (Sigma), 0.1 mM non-essential amino acid (Gibco) and the like.

Preferably, when the cell is mouse embryonic stem cell, the medium for culturing cell is the DMEM containing 15% FBS (fetal calves serum, Gibco) supplemented with 1000 U LIF (leukaemia inhibitory factor, Chemicon), 2 mM glutamine (Sigma), 1 mM sodium pyruvate (Sigma), and 0.1 mM β-mercaptoethanol (Sigma), 0.1 mM non-essential amino acid (Gibco) and the like.

Preferably, in step 4), the upstream primer for the PCR amplification reaction is an upstream primer for specific amplification of miRNA, the downstream primer is a universal primer (Universal Adaptor PCR Primer). Preferably, the universal primer is a universal primer with the trade name of AOMD-Q050, GeneCopoeia.

Preferably, the upstream primer for specific amplification of miRNA is an upstream primer for specific amplification of miRNA-292-3P, miRNA-294-3P, miRNA-323-3P or miRNA-21-5P.

Preferably, the upstream primer for specific amplification of miRNA-292-3P is a primer with the trade name of MmiRQP0944, GeneCopoeia.

Preferably, the upstream primer for specific amplification of miRNA-294-3P is a primer with the trade name of MmiRQP0947, GeneCopoeia.

Preferably, the upstream primer for specific amplification of miRNA-323-3P is a primer with the trade name of MmiRQP0410, GeneCopoeia.

Preferably, the upstream primer for specific amplification of miRNA-21-5P is a primer with the trade name of HmiRQP0316, GeneCopoeia.

Preferably, step 1) is carried out by the method comprising the steps of: collecting 5-10 ml of the medium in which the cells have been cultured overnight, centrifuging at 2000-3000 rpm to get the supernatant, and filtering the supernatant with a 0.45 µm filter.

Preferably, in step 1), 5 ml of the medium in which the cells have been cultured overnight is collected and centrifuged at 2000 rpm.

Preferably, in step 2), RNAs are extracted with a TRIZOL LS reagent.

Preferably, in step 3), the reverse transcription reaction is performed with the All-in-One™ miRNA reverse transcription kit.

In another aspect, the present invention provides a fast, dynamic, and non-invasive detection method for determining the pluripotency level of stem cells, and the method is performed by the above method to detect the expression levels of miRNAs in the medium of stem cells.

In yet another aspect, the present invention provides a fast, dynamic, and non-invasive detection method for determining the types and status of cells, and the method is performed by the above method to detect the expression levels of miRNAs in the cell culture media.

In still another aspect, the present invention provides a fast, dynamic, non-invasive detection method for identifying the status and properties of cells before clinical treatment, and the method is performed by the above method to detect the expression levels of miRNAs in the cell culture media.

Therefore, from a technical perspective, the invention provides a method for detecting the contents of miRNAs in the cell media, and demonstrates for the first time that the relative abundance of miRNAs in the cell media is consistent with that of miRNAs in the cultured cells. Thus, the types and status of cells can be determined by detecting the expression level of miRNA in the cell medium so as to avoid causing damages to cells. The method is especially suitable for the fundamental researches and clinical applications in which the number of cells is limited. From the perspective of the application, the following detections can be achieved by detecting the expression of miRNAs in the cell mediums: 1) the detection of the pluripotency levels of various types of stem cells; 2) the detection of the pluripotency levels of iPS cells in different induction stages; 3) the detection of the types and status of the cells derived by transdifferentiation and during transdifferentiation; 4) the detection of the status of the cells obtained by other means to change status of cells or the cells with other origins and types. A consistent result is observed by the inventor in the cultured cells of mice and human beings, demonstrating that there is no species limit for the present detection method. It can be used in the cells derived from any species. In summary, the present invention provides a method that effectively extracts miRNA from a cell medium and performs quantitative PCR detection to the extracted miRNAs to determine the types and status of cells. The method of the invention has achieved for the first time the fast determination of the cells type and status in the molecular level without destructing the cells. The method has great application value for monitoring the change of types of cells dynamically, early and fast screening of target cells, and monitoring the status of cells before clinical application.

### Brief Description of the Drawings

The examples of the invention are described in detail as follows in connection with drawings, wherein:

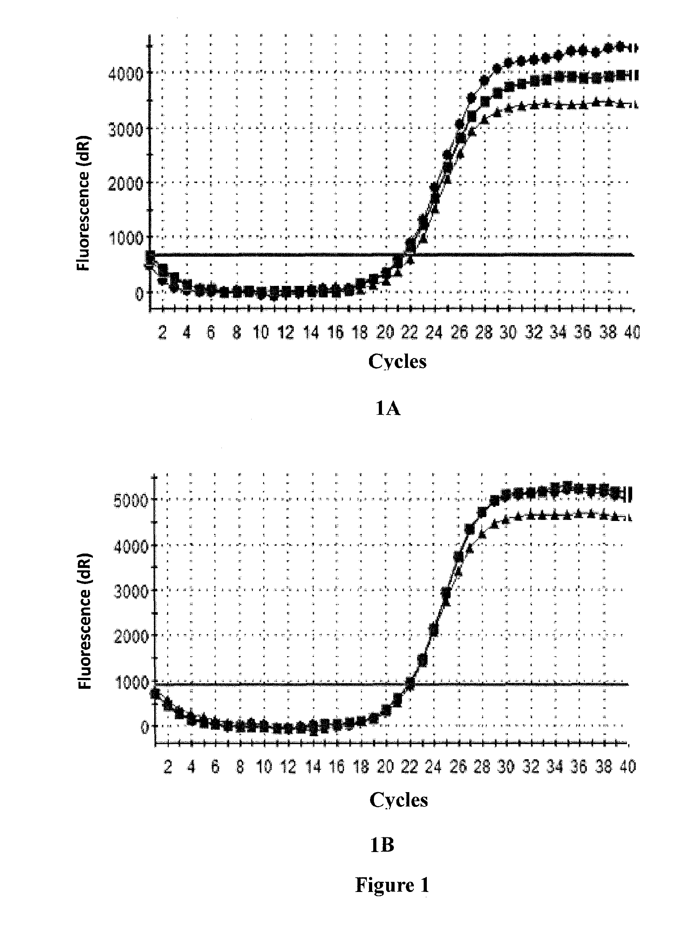

Figure 1 is the curves showing the amplification of miRNA-292-3P and miRNA-294-3P in the medium for mouse CL11 cells detected by PCR according to the method of the invention in Example 1, wherein Figure 1A is the curves showing three repeated amplification of miRNA-292-3P in CL11 cell medium, Figure 1B is the curves showing three repeated amplification of miRNA-294-3P in CL11 cell medium; in Figure 1, dR represents the baseline corrected fluorescence value;

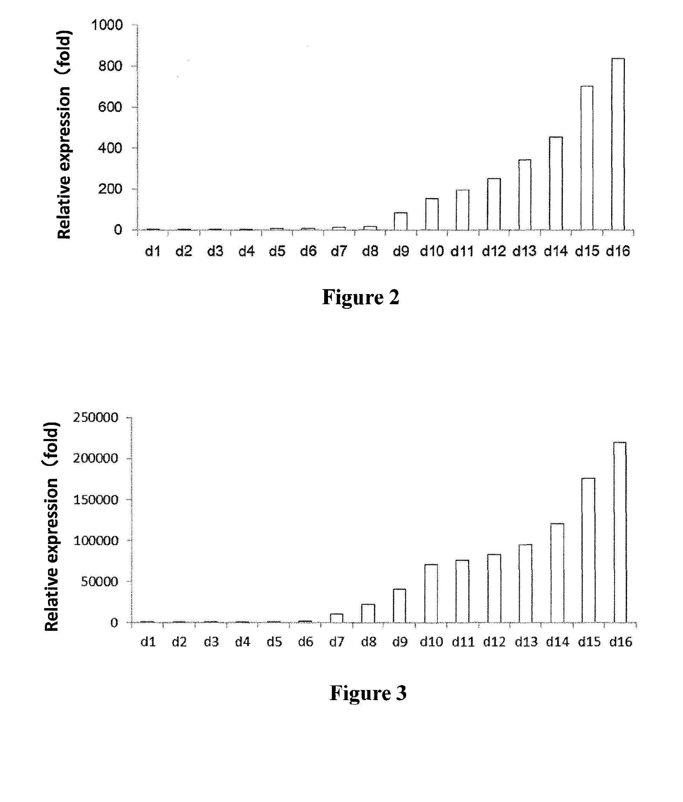

Figure 2 is the expression results of miRNA-294-3P in cell medium during the induction of mouse iPS cells detected by PCR according to the method of the invention in Example 2, wherein the ordinate represents the relative expression level of miRNA-294-3P in cell medium every day during the induction of iPS cells with the expression level of miRNA on day 1 as a baseline;
Figure 3 is the expression results of miRNA-294-3P in cells during the induction of mouse iPS cells detected by PCR amplification according to the method of the invention in Example 3, wherein the ordinate represents the expression level of miRNA-294-3P in cells every day during the induction of iPS cells with the expression level of miRNA on day 1 as a baseline;

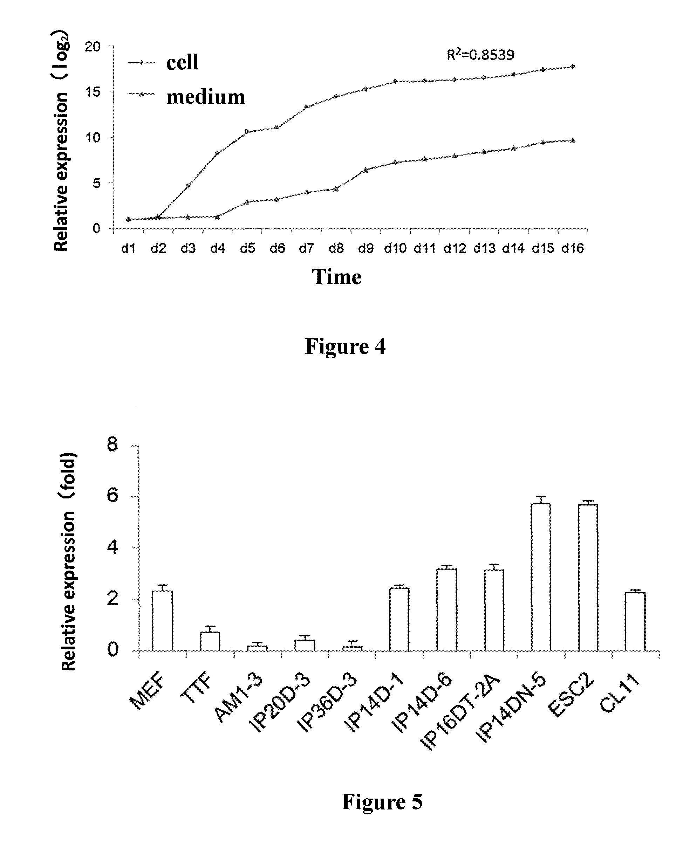

Figure 4 is the correlation analysis of the expression results of miRNA-294-3P in cells and in the corresponding medium during the induction of mouse iPS cells detected by PCR according to the method of the invention in Example 4;
Figure 5 is the expression results of miRNA-323-3P in the media for different cell lines detected by PCR amplification according to the method of the invention in Example 5, wherein the ordinate represents the expression level of miRNA-323-3P in different cell media with the expression level of miRNA in MEF cell medium as a baseline;

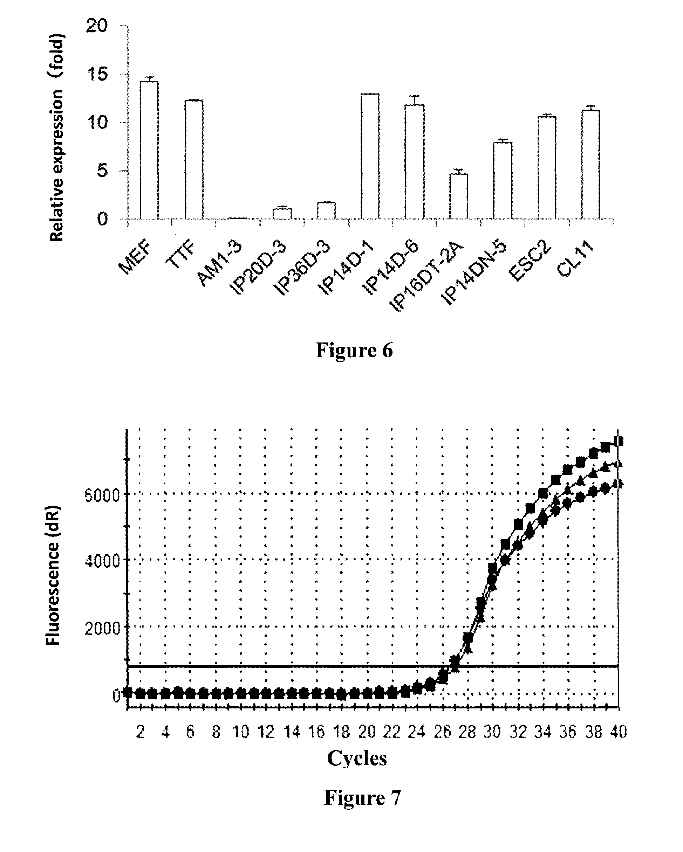

Figure 6 is the expression results of miRNA-323-3P in different cell lines detected by PCR according to the method of the invention in Example 6, wherein the ordinate represents the expression level of miRNA-323-3P in different cells with the expression level of miRNA in MEF cells as a baseline;
Figure 7 is the curve showing the amplification of miRNA-21-5P in the human breast cancer MCF-7 cell medium detected by PCR according to the method of the invention in Example 7; in the Figure, R represents the baseline corrected fluorescence value.

### Best Mode for Carrying out the Invention

It can be understood that the specific embodiment described herein is presented by way of illustration, rather than a limitation to the present invention. The major characters of the present invention can be used in various embodiments without departing the scope of the invention. A person skilled in the art will be able to confirm that many equivalents can be used in the specific steps described herein just by conventional experiments. It is considered that these equivalents are in the scope of the invention and covered by the claims.

Unless indicated specifically, MEF, TTF, AM1-3, IP20D-3, IP36D-3, IP14D-1, IP14D-6, IP16DT-2A, IP14DN-5, ESC2 and human breast cancer cell line MCF-7 in the following examples are all purchased from Institute of Zoology, Chinese Academy of Sciences, and CL11 cells are purchased from National Institute of Biological Sciences, Beijing.

Among them, the formulations of the medium for MEF, TTF and MCF-7 cells are 450 ml DMEM supplemented with 50 ml FBS and 5 ml 100x mycillin. The AM1-3 medium is the N₂B₂₇ medium supplemented with EGF and bFGF (their concentrations are all 20 ng/ml). The medium for mouse ES cell (embryonic stem cell) is the DMEM containing 15% FBS (fetal calves serum, Gibco), supplemented with 1000 U LIF (leukaemia inhibitory factor, Chemicon), 2 mM glutamine (Sigma), 1 mM sodium pyruvate (Sigma), and 0.1 mM β-mercaptoethanol (Sigma), 0.1 mM non-essential amino acid (Gibco) and the like. The medium for mouse iPS cell (induced pluripotent stem cells) is the DMEM/F12 containing 20% KOSR (serum substitute, Gibco) supplemented with 1000 U LIF (leukaemia inhibitory factor, Chemicon), 2 mM glutamine (Sigma), 1 mM sodium pyruvate (Sigma), and 0.1 mM β-mercaptoethanol (Sigma), 0.1 mM non-essential amino acid (Gibco) and the like.

Unless indicated specifically, the model of the fluorescence quantitative PCR instrument used in the following examples is Stratagene Mx 3000P fluorescence quantitative PCR instrument, purchased from Genetimes Technology, Inc.

Unless indicated specifically, the reagents used in the following examples are all analytically pure reagents, and commercially available.

### Example 1

a. collecting medium: 5 ml CL 11 cell medium in which the mouse embryonic stem cells have been cultured overnight is collected, centrifuged at 2000 rpm for 5 min to get the supernatant, and the supernatant is filtered with 0.45 µm filter.
b. extracting RNAs: after 15 ml TRIZOL LS reagent (Invitrogen) is added, 5 ml the collected medium is shaken vigorously for mixing homogeneously, and left to stand in ice for 15 min, and then shaken vigorously for mixing homogeneously after 4 ml chloroform is added and left to stand for 15 min. After centrifuged at 12000 g for 15 min, the supernatant is collected. After equivalent volume of isopropanol and 1 µl glycogen are added to precipitate for 1 h (-20°C), the supernatant is centrifuged at 12000 g for 20 min to precipitate the total RNAs. The precipitate is washed with 75% of ethanol, dried for 10 min, and dissolved in 20 µl RNAase free water, and then measured for the concentration of RNAs, and stored at -80°C for later use.
c. RNA reverse transcription: the reverse transcription reaction is performed on the RNAs obtained in step b using the All-in-One™ miRNA reverse transcription kit (GeneCopoeia) according to the instruction of the kit: adding 18 µl the RNAs obtained in step b into a 0.5 ml centrifugal tube, then adding 5 µl reverse transcription reaction solution, 1 µl PolyA polymerase, 1 µl RTase mix, and reacting at 37°C for 1 h, and at 85°C for 5 min to obtain a cDNA solution.
d. PCR detection: The PCR amplification reaction is performed on the cDNA solution obtained in step c using SYBR Green PCR detection kit. The upstream primers used are as follows: the upstream primer for specific amplification of miRNA-292-3P is the primer with trade name MmiRQP0944, GeneCopoeia; the upstream primer for specific amplification of miRNA-294-3P is the primer with trade name MmiRQP0947, GeneCopoeia; the upstream primer for specific amplification of small RNA U6 is the primer with trade name MmiRQP9002, GeneCopoeia; the downstream universal primer is Universal Adaptor PCR Primer (trade name: AOMD-Q050, GeneCopoeia). 1 µl each upstream primer and downstream primer, 9 µl the cDNA solution, 10 µl SYBR Green PCR reaction buffer are added into a fluorescence quantitative PCR reaction tube. The reaction conditions are as follows: predenaturation at 95 °C for 10 min, and then at 95°C for 30 s, at 60°C for 1 min, totally 40 cycles. Then the detection is performed with a fluorescence quantitative PCR instrument according to the instructions of the detection kit and the fluorescence quantitative PCR instrument, and the embryonic stem cell CL11 medium is amplified for three times in parallel.

The ΔCt value of the miRNA can be calculated according to the equation ΔCt=Ct_{miRNA}-Ct_{U6} by detecting the Ct values of the miRNA and small RNA U6 (internal reference value) in different cell media. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The amplification result of miRNA-292-3P (SEQ ID NO:1: AAAGUGCCGCCAGGUUUUGAGUGU) is shown in Figure 1A. The figure shows the curve indicating the amplification obtained from three amplifications of miRNA-292-3P in embryonic stem cell CL11 media in parallel. The Ct values that can be obtained from three amplifications of miRNA-292-3P in parallel from amplification curve are: 21.92, 21.81 and 21.99, respectively. The amplification results of the miRNA-294-3P (SEQ ID NO:2: AAAGUGCUUCCCUUUUGUGUGU) is shown in figure 1B: the figure shows the curves indicating the amplification obtained from three amplifications of miRNA-294-3P in embryonic stem cell CL11 media in parallel. The Ct values that can be obtained from three amplifications of miRNA-294-3P: 21.31, 21.49 and 22.15, respectively. It is demonstrated by the above results that there are miRNAs in cell medium, which is consistent the present detection method.

The PCR products obtained in the above Example are sent to Beijing Institute of Genomics, Chinese Academy of Sciences for sequencing by Sanger sequencing method. The results demonstrate that the obtained PCR product is target miRNA-292-3P.

### Example 2

a. collecting medium: about 1x10⁴ MEF cells are inoculated; 4 pluripotent factors such as Oct4, Sox2, Klf4 and c-Myc are packaged with the KOSR system, i.e., a reverse transcription virus; the induction of iPS cells is performed by virus solution infected MEF cells; 5 ml medium is collected every day from day 1 to day 16 during the induction of MEF into iPS cells; the collected medium is centrifuged at 2000 rpm for 5 min to get the supernatant, and the supernatant is filtered with 0.45 µm filter.
b. extracting RNAs: after 15 ml TRIZOL LS reagent (Invitrogen) is added, 5 ml the isolated medium is shaken vigorously for mixing homogeneously and left to stand in ice for 15 min, and then shaken vigorously for mixing homogeneously after 4 ml chloroform is added and left to stand for 15 min. After centrifuged at 12000g for 15 min, the supernatant is collected. After equivalent volume of isopropanol and 1 µl glycogen are added to precipitate for 1 h (-20°C), the solution is centrifuged at 12000g for 20 min to precipitate the total RNAs. The precipitate is washed with 75% of ethanol, dried for 10 min, and dissolved in 20 µl RNAase free water, and then measured for the concentration of the RNAs, and stored at-80°C for use later.
c. RNA reverse transcription: the reverse transcription reaction is performed on the RNAs obtained in step b using the All-in-One™ miRNA reverse transcription kit (GeneCopoeia) according to the instruction of the kit: adding 18 µl RNAs obtained in step b into a 0.5 ml centrifugal tube, then adding 5 µl reverse transcription reaction solution, 1 µl PolyA polymerase, 1 µl RTase mix, reacting at 37°C for 1 h, at 85°C for 5 min to obtain a cDNA solution.
d. PCR detection: The PCR amplification reaction is performed on the cDNA solution obtained in step c using the SYBR Green PCR detection kit. The upstream primers used are as follows: the upstream primer for specific amplification of miRNA-294-3P is the primer with the trade name of MmiRQP0947, GeneCopoeia; the upstream primer for specific amplification of small RNA U6 is the primer with the trade name of MmiRQP9002, GeneCopoeia; the downstream universal primer is an Universal Adaptor PCR Primer (trade name: AOMD-Q050, GeneCopoeia). 1 µl each upstream primer and downstream primer, 9 µl the cDNA solution, 10 µl SYBR Green PCR reaction buffer are added into a fluorescence quantitative PCR reaction tube. The reaction conditions are as follows: predenaturation at 95°C for 10 min, and then at 95°C for 30 s, at 60°C for 1 min, totally 40 cycles. Then the detection is performed with a fluorescence quantitative PCR instrument according to the instructions of the detection kit and the fluorescence quantitative PCR instrument.

The ΔCt value of the miRNA can be calculated according to the equation ΔCt=Ct_{miRNA}-Ct_{U6} by detecting the Ct value of the miRNA and small RNA U6 in cell media at different induction days. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The relative expression level of miRNA-294-3P (SEQ ID NO: 2: AAAGUGCUUCCCUUUUGUGUGU) in the medium is shown in figure 2. The figure shows the relative expression level of miRNA-294-3P in each sample obtained after the calculation of the fluorescence quantitative PCR results by the above equation. The ordinate represents the relative expression level of miRNA. It can be seen that miRNA-294-3P is a marker for ES cells specific expression. Therefore, during the induction of iPS cells, with the increase of the induction time, ES clone increases gradually. The relative expression level of miRNA-294-3P in cell medium also increases with the increase of the induction time. In the figure, the expression level of the induction on day 1 is set as 1, the expression levels at other induction times are all relative expression levels obtained by comparing with the expression level on day 1.

The PCR products obtained in the above example are sent to Beijing Institute of Genomics, Chinese Academy of Sciences for sequencing by Sanger sequencing method. The results demonstrate that the obtained PCR products are target miRNA-294-3P.

### Example 3

a. collecting cells: about 1x10⁴ MEF cells are inoculated, and the induction of iPS cells is performed by KOSR system and cells are collected every day from day 1 to day 16 during the induction of MEF into iPS cells. The solutions are changed every day, and cells are washed once with ice PBS.
b. extracting RNAs: after about 1x10⁴ cells are added, 1 ml TRIZOL reagent (Invitrogen) is shaken vigorously for mixing homogeneously, left to stand in ice for 15 min, and shaken vigorously for mixing homogeneously after 200 µl chloroform is added and left to stand for 15 min. After centrifuged at 12000 g for 15 min, the supernatant is collected. After the equivalent volume of isopropanol is added for precipitating for 1h (-20°C), the supernatant is centrifuged at 12000 g for 20 min to precipitate the total RNAs. The precipitate is washed with 75% of ethanol, dried for 10 min, dissolved in 40 µl RNAase free water, and measured for the concentration of the RNAs, stored at-80°C for use later.
c. RNA reverse transcription: the reverse transcription reaction is performed on the RNAs obtained in step b using the All-in-One™ miRNA reverse transcription kit (GeneCopoeia) according to the instruction of the kit: adding 18 µl the RNAs obtained in step b into a 0.5 ml of centrifugal tube, then adding 5 µl reverse transcription reaction solution, 1 µl PolyA polymerase, 1 µl RTase mix, reacting at 37°C for 1 h, at 85°C for 5 min to obtain a cDNA solution.
d. PCR detection: the PCR amplification reaction is performed on the cDNA solution obtained in step c using SYBR Green PCR detection kit. The upstream primer: the upstream primer for specific amplification of miRNA-294-3P is the primer with the trade name of MmiRQP0947, GeneCopoeia; the upstream primer for specific amplification of small RNA U6 is the primer with trade name of MmiRQP9002, GeneCopoeia. The downstream universal primer is a Universal Adaptor PCR Primer (trade name: AOMD-Q050, GeneCopoeia). 1 µl each upstream primer and downstream primer, 9 µl the cDNA solution, 10 µl SYBR Green PCR reaction buffer are added into a fluorescence quantitative PCR reaction tube. The reaction conditions are as follows: predenaturation at 95°C for 10 min, then at 95°C for 30 s, at 60°C for 1 min, totally 40 cycles. Then the detection is performed with a fluorescence quantitative PCR instrument according to the instructions of the detection kit and the fluorescence quantitative PCR instrument.

The ΔCt value of the miRNA can be calculated according to the equation ΔCt=Ct_{miRNA}-Ct_{U6} by detecting the Ct values of the miRNA and small RNA U6 in different cells. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The relative expression level of miRNA-294-3P in cells is shown in Figure 3. The figure shows the relative expression level of miRNA-294-3P in each sample which is obtained after the calculation of the fluorescence quantitative PCR results according to the above equation. The ordinate represents the relative expression level of the miRNA. It can be seen that during the induction of iPS cells, the relative expression level of miRNA-294-3P in cells increases with the increase of the induction time. The results indicate that the expression level of the induction on day 1 is set as 1, the expression levels at the other induction time are all relative expression levels obtained by comparing with the expression level on day 1.

The miRNA-294-3P (SEQ ID NO 2: AAAGUGCUUCCCUUUUGUGUGU) is a marker for ES cells specific expression. Therefore, during the induction of iPS cells, with the increase of the induction time, ES cell clone increases gradually; the relative expression level of miRNA-294-3P in cells increases with the increase of the induction time. As compared to Figure 2, it is demonstrated that the expression level of miRNA-294-3P in cell medium is consistent with the change tendency of states of the cultured cells.

The PCR products obtained in the above Example are sent to Beijing Institute of Genomics, Chinese Academy of Sciences for sequencing by Sanger sequencing method. The results demonstrate that the obtained PCR products are target miRNA-294-3P.

### Example 4

The correlation between the results detected in the above Examples 2 and 3 is analyzed. In order to show the results better, the ordinate is the log₂ value of the expression level as shown in Figure 4. We have made a regression analysis on the expression level of the miRNAs detected in medium and in cells on day 3 to day 16, and the following regression equation is obtained for further statistical analysis: log₂E_{cells} = 1.5268× log₂E_{medium} + 3.1026, wherein R²=0.8539. The analysis result demonstrates that above 85% of data of the detected results in cells and the detected results in medium can be described with the regression equation. The F distribution is used to test the equation, wherein P = 3.181e-07, demonstrating that the correlation between the expression levels of miRNAs detected in medium and in cells is very significant.

In order to verify the regression equation, we repeated the assay twice according to the method. The correlation coefficients R² is obtained by fitting the expression level of miR-294 in cells and the expression level of miR-294 actually detected in cells via the regression equation using the expression level of miR-294 in medium are 0.8234 and 0.8296, respectively; P =1.218e-06 and 9.487e-07. The results demonstrate that the regression equation can be better repeated on data 2 and 3.

Accordingly, the equation can be used for the correlation between the expression of miRNAs in medium and the expression of miRNAs in cells every day from day 3 to day 16 during the iPS induction on MEF cells using reverse transcription virus in KOSR system.

### Example 5

a. collecting medium: 5 ml of the media in which MEF, TTF, AM1-3, IP20D-3, IP36D-3, IP14D-1, IP14D-6, IP16DT-2A, IP14DN-5, ESC2 and CL11 cells have been cultured overnight are collected respectively, centrifuged at 2000 rpm for 5 min to get the supernatant. The supernatant is filtered with 0.45 µm filter.
b. extracting RNAs: after 15 ml TRIZOL LS reagent (Invitrogen) added, 5 ml the collected different cell media is shaken vigorously for mixing homogeneously, left to stand in ice for 15 min, then shaken vigorously for mixing homogeneously after 4 ml chloroform is added and left to stand for 15 min. After centrifuged at 12000g for 15 min, the supernatant is collected. After equivalent volume of isopropanol and 1 µl glycogen are added to precipitate for 1h (-20°C), the solution is centrifuged at 12000g for 20 min to precipitate the total RNAs. The precipitate is washed with 75% of ethanol, dried for 10 min, dissolved in 20 µl RNAase free water, and measured for the concentration of the RNAs, and stored at-80°C for use later.
c. RNA reverse transcription: The reverse transcription reaction is performed on the RNAs obtained in step b using the All-in-One™ miRNA reverse transcription kit (GeneCopoeia) according to the instruction of the kit: adding 18 µl of the RNAs obtained in step b into a 0.5 ml of a centrifugal tube, then adding 5 µl reverse transcription reaction solution, 1 µl PolyA polymerase, 1 µl RTase mix, reacting at 37°C for 1 h, at 85°C for 5 min to obtain a cDNA solution.
d. PCR detection: The PCR amplification reaction is performed on the cDNA solution obtained in step c using SYBR Green PCR detection kit. The upstream primer: the upstream primer for specific amplification of the miRNA-323-3P is the primer with the trade name of MmiRQP0410, GeneCopoeia, the upstream primer for specific amplification of small RNA U6 is the primer with the trade name of MmiRQP9002, GeneCopoeia. The downstream universal primer is a Universal Adaptor PCR Primer (trade name: AOMD-Q050, GeneCopoeia). 1 µl each upstream primer and downstream primer, 9 µl the cDNA solution, 10 µl SYBR Green PCR reaction buffer are added into a fluorescence quantitative PCR reaction tube. The reaction conditions are as follows: predenaturation at 95°C for 10 min, then at 95°C for 30 s, at 60°C for 1 min, totally 40 cycles. Then detection is performed with a fluorescence quantitative PCR instrument according to the instructions of the detection kit and the fluorescence quantitative PCR instrument.

The ΔCt value of miRNA can be calculated according to the equation ΔCt=Ct_{miRNA}-Ct_{U6} by detecting ΔCt values of the miRNA and small RNA U6 in different cell media. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The relative expression level of miRNA-323-3P (SEQ ID NO 3: CACAUUACACGGUCGACCUCU) is shown in Figure 4. The figure shows the relative expression level of miRNA-323-3P in each cell media obtained after the calculation of the fluorescence quantitative PCR results according to the above equation. The ordinate represents the relative expression level of miRNA. It can be seen that miRNA-323-3P is a marker for stem cell pluripotency level, which has high expression in cells with high pluripotency level. Therefore, miR-323-3P has high expression in the media of fibroblast (MEF, TTF) and 4N-iPS cell (IP14D-1, IP14D-6, IP16DT-2A, IP14DN-5) and ES cell (ESC2, CL11), but there is very low or no expression of miR-323-3P in the media of 2N-iPS cell (IP20D-3, IP36D-3) and neuronal precursor cell (AM1-3). In the Figure, the expression level in MEF cell medium is set as 1, the expression levels in other cells are all relative expression level obtained as compared to that of in MEF cells.

The PCR products obtained in the above Example are sent to Beijing Institute of Genomics, Chinese Academy of Sciences for sequencing by Sanger sequencing method. The results demonstrate that the obtained PCR products are target miRNA-323-3P.

### Example 6

a. collecting cells: about 1x10⁶ of MEF, TTF, AM1-3, IP20D-3, IP36D-3, IP14D-1, IP14D-6, IP16DT-2A, IP14DN-5, ESC2 and CL11 cells are collected respectively, and washed once with ice PBS.
b. extracting RNAs: after 1 ml TRIZOL reagent (Invitrogen) added, the different cells are shaken vigorously for mixing homogeneously, left to stand in ice for 15 min, and shaken vigorously for mixing homogeneously after 200 µl chloroform is added and left to stand for 15 min. After centrifuged at 12000 g for 15 min, the supernatant is collected. After equivalent volume of isopropanol added to precipitate for 1h (-20°C), the supernatant is centrifuged at 12000 g for 20 min to precipitate the total RNAs. The precipitate is washed by 75% ethanol, dried for 10 min, dissolved in 40 µl RNAase free water, measured for the concentration of the RNAs, and then stored at -80°C for later use.
c. RNA reverse transcription: The reverse transcription reaction is performed on the RNAs obtained in step b using the All-in-One™ miRNA reverse transcription kit (GeneCopoeia) according to the instruction of the kit: adding 18 µl RNAs obtained in step b into a 0.5 ml centrifugal tube, then adding 5 µl reverse transcription reaction solution, 1 µl PolyA polymerase and 1 µl RTase mix, reacting at 37°C for 1 h and then at 85°C for 5 min to obtain a cDNA solution.
d. PCR detection: The PCR amplification reaction is performed on the cDNA solution obtained in step c using SYBR Green PCR detection kit. The upstream primer for amplification is the upstream primer for specific amplification of miRNA-323-3P and U6, the upstream primer for miRNA-323-3P is the primer with the trade name of MmiRQP0410, GeneCopoeia, the upstream primer for specific amplification of the small RNA U6 is the primer with the trade name of MmiRQP9002, GeneCopoeia and the downstream universal primer is an Universal Adaptor PCR Primer (trade name: AOMD-Q050, GeneCopoeia). 1 µl each upstream primer and downstream primer, 9 µl the cDNA solution, 10 µl SYBR Green PCR reaction buffer are added into a fluorescence quantitative PCR reaction tube, respectively. The reaction conditions are as follows: predenaturation at 95°C for 10 min, then at 95°C for 30 s, at 60°C for 1 min, totally 40 cycles. Then the detection is performed with a fluorescence quantitative PCR instrument according to the instructions of the detection kit and the fluorescence quantitative PCR instrument.

The ΔCt value of the miRNA can be calculated according to the equation ΔCt=CtmiRNA-CtU6 by detecting the Ct values of the miRNA and the small RNA U6 in different cells. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The relative expression level of miRNA-323-3P (SEQ ID NO 3: CACAUUACACGGUCGACCUCU) in each cells is shown in Figure 5. The figure shows the relative expression level of miRNA-323-3P in each cell calculated from the fluorescence quantitative PCR result according to above equation. The ordinate represents the relative expression level of the miRNA. It can be seen that miRNA-323-3P has a high expression in fibroblast (MEF, TTF) and 4N-iPS cell (IP14D-1, IP 14D-6, IP16DT-2A, IP 14DN-5) and ES cell (ESC2, CL11), but expresses in very low level or does not express in 2N-iPS cell (IP20D-3, IP36D-3) and neuronal precursor cell (AM1-3). Consistent with the expression trend in the corresponding cell medium, in the figure, the expression level in MEF cells is set as 1, and the expression levels in other cells are all the relative expression levels obtained as compared to that of in MEF cells.

The PCR products obtained in the above Example are sent to Beijing Institute of Genomics, Chinese Academy of Sciences for sequencing by Sanger sequencing method. The results demonstrate that the obtained PCR products are target miRNA-323-3P.

### Example 7

a. collecting medium: 5 ml of the medium in which human breast cancer cell line MCF-7 has been cultured overnight is collected, centrifuged at 2000 rpm for 5 min to get the supernatant, and the supernatant is filtered with a 0.45 µm filter.
b. extracting RNAs: after 15 ml TRIZOL LS reagent (Invitrogen) is added, 5 ml the collected medium is shaken vigorously for mixing homogeneously, left to stand in ice for 15 min, and then shaken vigorously for mixing homogeneously after 4 ml chloroform is added and left to stand for 15 min. After centrifuged at 12000 g for 15 min, the supernatant is collected. After equivalent volume of isopropanol and 1 µl glycogen are added to precipitate for 1 h (-20°C), the solution is centrifuged at 12000g for 20 min to precipitate the total RNAs. The precipitate is washed with 75% of ethanol, dried for 10 min, dissolved in 20 µl RNAase free water, and then measured the concentration of the RNAs, and stored at -80°C for later use.
c. RNA reverse transcription: The reverse transcription reaction is performed on the RNA obtained in step b using the All-in-One™ miRNA reverse transcription kit (GeneCopoeia) according to the instruction of the kit: adding 18 µl the RNAs obtained in step b into a 0.5 ml centrifugal tube, then adding 5 µl reverse transcription reaction solution, 1 µl PolyA polymerase, 1 µl RTase mix, reacting at 37°C for 1 h, at 85°C for 5 min to obtain a cDNA solution.
d. PCR detection: The PCR amplification reaction is performed on the cDNA solution obtained in step c using SYBR Green PCR detection kit. The upstream primer: the upstream primer for specific amplification of the miRNA-21-5P is the primer with the trade name of HmiRQP0316, GeneCopoeia, the upstream primer for specific amplification of small RNA U6 is the primer with the trade name of MmiRQP9002, GeneCopoeia, and the downstream universal primer is a Universal Adaptor PCR Primer (trade name AOMD-Q050, GeneCopoeia). 1 µl each upstream primer and downstream primer, 9 µl cDNA solution, 10 µl SYBR Green PCR reaction buffer are added into a fluorescence quantitative PCR reaction tube. The reaction conditions are as follows: predenaturation at 95°C for 10 min, then at 95°C for 30 s, at 60°C for 1 min, totally 40 cycles. Then detection is performed with a fluorescence quantitative PCR instrument according to the instructions of the detection kit and the fluorescence quantitative PCR instrument. The amplification of human breast cancer cell MCF-7 cell medium is performed for three times in parallel.

The ΔCt value of the miRNA can be calculated according to the equation ΔCt=Ct_{miRNA}-Ct_{U6} by detecting the ΔCt values of the miRNA and the small RNA U6 in cell medium. The relative expression level of the miRNA can be determined according to the ΔCt value. The lower the ΔCt value is, the higher the expression level of the corresponding miRNA is; while the larger the ΔCt value is, the lower the corresponding expression level of the miRNA is.

The amplification result of miRNA-21-5P (SEQ ID NO 4: UAGCUUAUCAGACUGAUGUUGA) is shown in Figure 6. The figure shows the curve indicating the amplification of human breast cancer cell MCF-7 medium obtained by amplifying miRNA-21-5P three times in parallel. The Ct values for three amplifications in parallel from the amplification curve are 27.01, 26.98 and 27.35, respectively. The above results demonstrate that there is miRNA in human breast cancer cell MCF-7 cell medium, which can be consistently detected by the present method.

The PCR products obtained in the above Example are sent to Beijing Institute of Genomics, Chinese Academy of Sciences for sequencing by Sanger sequencing method. The results demonstrate that the obtained PCR products are target miRNA-21-5P.

The present invention relates to the ES cells specific highly expressed miRNA marker miRNA-292-3P and miRNA-294-3P, as well as miRNA-323-3P in *Dlk1-Dio3* imprinted region, wherein the miRNA in *Dlk1-Dio3* imprinted region is low expressed in 2N-iPS cells while highly expressed in 4N-iPS cells. It can be seen from our results: 1. during the induction of MEF into iPS cells, MEF is fibroblast without the expression of miRNA-294-3P, and miRNA-294-3P is specifically expressed in ES cells. With the increase of the induction time, there are increasing numbers of iPS cells. The expression of miRNA-294-3P gradually increases, and then the transformation of cells fate is observed in time; 2. miRNA-323-3P has high expression in fibroblasts (MEF, TTF), 4N-iPS cells (IP14D-1, IP14D-6, IP16DT-2A, IP14DN-5) and ES cells (ESC2, CL11), and a very low or no expression in 2N-iPS cells (IP20D-3, IP36D-3) and neuronal precursor cells (AM1-3). The pluripotency levels of cells can be identified by detecting the expression of the miRNA in medium; 3. The miRNA can also be detected in human cell medium, demonstrating that the method is suitable for multiple species. It provides a better foundation for later use of the method in the detection of properties of cells before clinical treatment with ES and iPS cells, the detection of properties of the cells derived by transdifferentiation, the detection of developmental potential before IVF embryo implant in clinical and other application that needs to non-invasive determination the status of cells. The miRNA in cell medium is detected for the first time by the method in the invention, and it is proved that the miRNA expression tendency in medium is consistent with that in cultured cells. It is proved for the first time that the type and status of cells can be identified by detecting the expression of miRNA in a medium without destroying cells. It is especially suitable for the fundamental research and clinical application where the number of cells is limited. In summary, it is used in the invention for the first time that a non-invasive method is used to detect the expression level of miRNA in cells with different types and status. The present invention provides a new means for dynamic non-destructive identification of type and properties of cells as well as observing transformation of cell fate.

## Claims

1. A method for fast, conveniently and non-invasively detecting the expression level of miRNA in cells and determining the types and status of the cells, which is achieved by detecting the type and expression level of miRNAs in cell culture media; preferably, the detection of the expression level of miRNAs in the media is carried out by the method comprising the following steps:
1) collecting the medium in which cells have been cultured overnight, centrifuging the collected medium to get the supernatant, and filtering the supernatant;
2) extracting RNAs from the filtered supernatant obtained in step 1);
3) performing a reverse transcription reaction using the RNAs extracted in step 2) to obtain a cDNA solution;
4) performing a PCR amplification reaction using the cDNA solution obtained in step 3), and then detecting the PCR products with a fluorescence quantitative PCR instrument so as to obtain the expression level of miRNAs in the medium;
5) determining the change degree of the expression level of miRNAs in cells according to the change degree of the expression level of miRNAs in the medium;
preferably, plotting the changing curve of the expression level of miRNAs in the medium and calculating the amount of miRNAs in cells;
preferably, the medium in which the cells have been cultured overnight is the medium in which the cells have been cultured for 3-16 days.

2. The method according to claim 1, **characterized in that** when detecting the expression level of miRNA in cells during iPS induction, the cells are cultured and then the expression level of miRNAs in the medium is measured, and the expression level of miRNAs in cells is calculated according to the equation log₂E _{cells} = 1.5268× log₂E _{medium} + 3.1026 or similar equations obtained by studying the properties of the cells; preferably, the cells are those that have been cultured for 0-16 days; more preferably, the cells are those that have been cultured for 3-16 days.

3. The method according to claim 1 or 2, **characterized in that** in step 1), the cells are one or more selected from the group consisting of human or animal embryo fibroblast, human or animal induced pluripotent stem cell, animal embryonic stem cell, human or animal adult stem cell, human or animal cell derived by transdifferentiation, differentiated cell of human or animal, tumor cell or diseased stem cell;
preferably, the animal induced pluripotent stem cells are selected from 2N-iPS cells and 4N-iPS cells; more preferably, 2N-iPS cells comprises IP20D-3 and IP36D-3 cell lines, 4N-iPS cells comprises cell lines such as IP14D-1, IP14D-6, IP16DT-2A and IP 14DN-5;
preferably, the animal embryonic stem cell is ESC2 and/or CL11;
preferably, the animal embryo fibroblast is mouse embryo fibroblast;
preferably, the differentiated cell of human or animals is selected from animal tail-tip cell and/or mouse neuronal precursor cell; more preferably, the animal tail-tip cell is mouse tail-tip cell; the neuronal precursor cell is mouse neuronal precursor cell;
preferably, the tumor cell is human breast cancer cell.

4. The method according to claim 3, **characterized in that** in step 1) when the cell is mouse embryo fibroblast, mouse tail-tip cell or human breast cancer cell, the medium for culturing cell is 450 ml DMEM medium supplemented with 50 ml FBS, 5 ml 100x mycillin; or
when the cell is mouse neuronal precursor cell, the medium for culturing cell is the N₂B₂₇ medium supplemented with EGF and bFGF; preferably, the concentrations of EGF and bFGF are all 20 ng/ml; or
when the cell is mouse iPS induced pluripotent stem cell, the medium for culturing cell is the DMEM/F12 medium containing 20% serum substitute; preferably, the DMEM/F12 medium further contains 1000 leukaemia inhibitory factor, 2 mM glutamine, 1 mM sodium pyruvate, and 0.1 mM β-mercaptoethanol, 0.1 mM non-essential amino acids;
when the cell is mouse embryonic stem cell, the medium for culturing cell is the DMEM medium containing 15% FBS; preferably, the DMEM medium further contains 1000 U leukaemia inhibitory factor, 2 mM glutamine, 1 mM sodium pyruvate, 0.1 mM β-mercaptoethanol and 0.1 mM non-essential amino acids.

5. The method according to any one of claims 1 to 4, **characterized in that** in step 4), the upstream primer for the PCR amplification reaction is an upstream primer for specific amplification of miRNA, the downstream primer is a universal primer; preferably, the universal primer is the universal primer with the trade name of AOMD-Q050, GeneCopoeia.

6. The method according to claim 5, **characterized in that** the upstream primer for specific amplification of miRNA is the upstream primer for specific amplification of miRNA-292-3P, miRNA-294-3P, miRNA-323-3P or miRNA-21-5P; preferably, the upstream primer for specific amplification of the miRNA-292-3P is the primer with the trade name of MmiRQP0944, GeneCopoeia; the upstream primer for specific amplification of the miRNA-294-3P is the primer with the trade name of MmiRQP0947, GeneCopoeia; the upstream primer for specific amplification of the miRNA-323-3P is the primer with the trade name of MmiRQP0410, GeneCopoeia; the upstream primer for specific amplification of the miRNA-21-5P is the primer of HmiRQP0316, GeneCopoeia.

7. The method according to any one of claims 1 to 6, **characterized in that** step 1) is carried out by the method comprising following steps: collecting 5-10 ml the medium in which cells have been cultured overnight, centrifuging at 2000-3000 rpm to get the supernatant, and filtering the supernatant; preferably, collecting 5 ml the medium in which cells have been cultured overnight, centrifuging at 2000 rpm; more preferably, filtering the supernatant with a 0.45 µm filter; more preferably, in step 2), the RNAs are extracted with TRIZOL LS reagent.

8. The method according to any one of claims 1 to 8, **characterized in that** in step 3), the reverse transcription reaction is performed using All-in-One™ miRNA reverse transcription kit.

9. A method for determining the types and status of cells and the pluripotency level of stem cells, which is suitable for the detection of pluripotency levels of various types of stem cells; the detection of the pluripotency levels of iPS cells in different induction stages; the detection of the types and status of cells generated by transdifferentiation and during transdifferentiation; and the detection of status of cells generated by other means to change status of cells or cells of other origins and types, **characterized in that** the method is performed by detecting the expression level of miRNAs in cells according to the method of any one of claims 1 to 8.

10. A method for identifying the types and status of cells before clinical treatment, which is suitable for the detection of properties of ES and iPS cells before clinical treatment, the detection of properties of cells derived by transdifferentiation, the detection of developmental potential before IVF embryo implant in clinical and other application that needs to non-invasively determine status of cells, **characterized in** the method is performed by detecting the expression level of miRNAs in cells according to the method of any one of claims 1 to 8.


<110>  INSTITUTE OF ZOOLOGY, CHINESE ACADEMY OF SCIENCES; \177INSTITUTE OF
GENETICS AND DEVELOPMENTAL BIOLOGY, CHINESE ACADEMY OF SCIENCES

<120>  METHOD FOR NONDESTRUCTIVE DETECTION OF MIRNA EXPRESSION IN CELL AND
DETERMINATION OF CELL TYPE AND STATE

<130>  PANBP/P57560EP

<140>   13822557.8
<141>   2013/05/20

<150>   CN 201210258472.8
<151>   2012/07/24

<150>   PCT/CN2013/075903
<151>   2013/05/20

<160>  4

<170>  PatentIn version 3.3

<210>  1
<211>  24
<212>  RNA
<213>  Mice

<400>  1
aaagugccgc cagguuuuga gugu                                              24


<210>  2
<211>  22
<212>  RNA
<213>  Mice

<400>  2
aaagugcuuc ccuuuugugu gu                                                22


<210>  3
<211>  21
<212>  RNA
<213>  Mice

<400>  3
cacauuacac ggucgaccuc u                                                 21


<210>  4
<211>  22
<212>  RNA
<213>  Homo sapiens

<400>  4
uagcuuauca gacugauguu ga                                                22